# EUROPEAN PATENT SPECIFICATION

(11) **EP 2 528 619 B1**
(45) Date of publication and mention of the grant of the patent: **04.12.2013**
(21) Application number: 11702413.3
(22) Date of filing: 26.01.2011
(51) Int. Cl.: A61K 38/44, G01N 33/50

(54) **SCREENING METHOD FOR IDENTIFYING COMPOUNDS WHICH BLOCK TUMOUR GROWTH BY INDUCING IRREVERSIBLE SENESCENCE IN TUMOUR CELLS**
SCREENING-VERFAHREN ZUR IDENTIFIZIERUNG VON VERBINDUNGEN, DIE DAS TUMORWACHSTUM DURCH INDUZIERUNG DER IRREVERSIBLEN SENESZENZ IN TUMORZELLEN BLOCKIEREN
PROCÉDÉ DE CRIBLAGE POUR IDENTIFIER DES COMPOSÉS QUI BLOQUENT LA CROISSANCE TUMORALE EN INDUISANT UNE SÉNESCENCE IRRÉVERSIBLE DANS DES CELLULES TUMORALES

(30) Priority: 26.01.2010 US 298315 P; 26.01.2010 EP 10305089
(43) Date of publication of application: 05.12.2012
(73) Proprietor: Centre Léon Bérard, 69008 Lyon (FR); Centre National de la Recherche Scientifique CNRS, 75016 Paris (FR); INSTITUT NATIONAL DE LA SANTE ET DE LA RECHERCHE MEDICALE (INSERM), 75013 Paris (FR)
(72) Inventor: BERNARD, David, 38460 Trept (FR); BARTHOLIN, Laurent, 69008 Lyon (FR); LELIEVRE, Etienne, 34090 Montpellier (FR); AUGERT, Arnaud, 69007 Lyon (FR)
(74) Representative: Regimbeau
(86) International application number: PCT/EP2011/051029
(87) International publication number: WO 2011/092181

(56) References cited:
- WO-A2-2004/014319
- WO-A2-2007/126457
- WO-A2-2009/017833
- LLEONART MATILDE E ET AL: "Senescence induction; a possible cancer therapy", MOLECULAR CANCER, BIOMED CENTRAL, LONDON, GB LNKD- DOI:10.1186/1476-4598-8-3, vol. 8, no. 1, 8 January 2009 (2009-01-08) , page 3, XP021050183, ISSN: 1476-4598
- EWALD J A ET AL: "Drug-induced senescence bystander proliferation in prostate cancer cells in vitro and in vivo", BRITISH JOURNAL OF CANCER, vol. 98, no. 7, April 2008 (2008-04), pages 1244-1249, XP002577030, ISSN: 0007-0920
- HUMBERT NICOLAS ET AL: "A Genetic Screen Identifies Topoisomerase 1 as a Regulator of Senescence", May 2009 (2009-05), CANCER RESEARCH, VOL. 69, NR. 10, PAGE(S) 4101-4106, XP002577031, ISSN: 0008-5472 the whole document
- ERLER JANINE T ET AL: "Lysyl oxidase is essential for hypoxia-induced metastasis", NATURE, NATURE PUBLISHING GROUP, LONDON, GB LNKD- DOI:10.1038/NATURE04695, vol. 440, no. 7088, 1 April 2006 (2006-04-01), pages 1222-1226, XP002508335, ISSN: 0028-0836 cited in the application
- ERLER JANINE T ET AL: "Hypoxia-induced lysyl oxidase is a critical mediator of bone marrow cell recruitment to form the premetastatic niche.", CANCER CELL 6 JAN 2009 LNKD- PUBMED:19111879, vol. 15, no. 1, 6 January 2009 (2009-01-06), pages 35-44, XP002625567, ISSN: 1878-3686 cited in the application
- BONDAREVA ALLA ET AL: "The Lysyl Oxidase Inhibitor, beta-Aminopropionitrile, Diminishes the Metastatic Colonization Potential of Circulating Breast Cancer Cells", PLOS ONE, vol. 4, no. 5, May 2009 (2009-05), XP002577029, ISSN: 1932-6203 cited in the application

## Description

A widely used strategy for blocking cancer development in clinic is to induce apoptotic cancer cell death through radiation and chemotherapeutic drugs. Recently, it has been demonstrated that these radiations or drugs also act by inducing a senescence response. This senescence response results in a permanent form of cell cycle arrest associated with the appearance of specific markers such as the Senescent Associated β Galactosidase activity or "SA-β-Gal" (Dimri et al., 1995). Cancer cells that escape these treatments may have escaped both apoptosis and senescence programs. Thus senescence, as apoptosis, can be considered as a failsafe program exerting anti-tumoral activity in cancer cells by preventing tumour initiation. Indeed, during the transformation process, normal cells escape senescence (Braig et al., 2005; Chen et al., 2005; Collado et al., 2005).

In this context, screening for compounds restoring the senescence response in tumour cells appears to be a new and attractive way to isolate new therapeutic drugs for treating cancers. A major problem in the efficiency of anti-tumour compounds targeting the senescence response is the potential resistance/escape of the treated cells. So far, no cellular models were reported for identifying compounds for which no spontaneous senescence escape occurs.

The present application is related to new methods for identifying compounds for treating cancers, which induce an irreversible senescence in human cells. New *in vitro* methods have been developed providing efficient screening for compounds that stabilize senescence and thus block senescence escape. These methods have identified a compound which inhibits senescence escape. This compound, aminopropionitrile (BAPN) is a well known inhibitor of Lysyl oxidase (LOX). It has been shown that Lysyl oxidase is essential for hypoxia-induced metastasis (Erler et al., 2006; Erler et al., 2009). Inhibitors of lysyl oxidase activity have also been proposed for reducing tumour growth and particularly metastatic tumour growth (WO 2007/126457). However, Erler *et al.* reports that there is no effect of LOX inhibition on primary tumour growth and so far LOX inhibition has been described to exert anti-metastatic effects with no effect over the primary tumors (Erler *et al.,* 2006; Erler *et al.,* 2009).

WO2009/017833 describes methods and compositions for treatment and diagnosis of fibrosis, tumor invasion, angiogenesis and metastasis. LOX inhibitors seem to have an inhibitory effect on epithelial-mesenchymal transition (EMT) which is involved in tumor invasion and metastasis. Treatment of primary tumors or of Pancreatic Ductal Adenocarcinoma is not described nor suggested.

Bondareva *et al.* (2009) shows that β-aminopropionitrile, a lysyl oxidase inhibitor, diminishes the metastatic colonization potential of circulating breast cancer cells. The authors conclude that LOX inhibition might be useful in metastasis prevention.

In order to investigate the effect of BAPN and to demonstrate that chemical compounds identified in the *in vitro* model of the present invention are also efficient *in vivo,* a mouse model of pancreatic cancers was used. Pancreatic Ductal Adenocarcinoma (PDAC), which represents the vast majority of all pancreatic cancers (more than 80 % of them), is a very aggressive tumor, always lethal, accounting for the fifth cause of death by cancer. It is particularly disturbing that despite progress made in the understanding of the genetics of pancreatic cancers, the overall survival rate of patients with this disease has not changed significantly in the last four decades. Hence, there is a crucial need to find new therapeutics to efficiently fight PDAC.

The first model of genetically engineered mice with PDAC was described in 2003. In this model, in agreement with human epidemiological data, the activation of Kras (Kras-G 12D mutant also called Kras-CA, "CA" standing for Constitutively Activated) induces the formation of precancerous ductal lesions (Hingorani *et al.,* 2003) but has to be combined with Ink4a/Arf deficiency to potently induce PDAC (Bardeesy *et al.,* 2001). To validate the anti-tumoral effect of BAPN *in vivo* we used Pdx1-Cre; Kras-CA^{lox/+}; Ink4a/Arf-KO^{lox/lox} genetically relevant modified mice.

We wanted to know, as our *in vitro* results suggested, whether or not the LOX inhibition by BAPN treatment could inhibit primary tumor growth. To this end we used a genetically modified mouse expressing a mutated K-RAS and knocked out for the Ink4A/Arf locus as described above. These mice develop aggressive pancreatic tumors in a few weeks after birth (Aguirre et al., 2003). To test a potential effect of Lox inhibition by BAPN, we treated these mice by intra peritoneal injection of vehicle or BAPN. 6-7 weeks after birth, mice were sacrificed and pancreatic tumors examined. As expected, the two control mice treated with "vehicle" showed numerous multifoci pancreatic tumors clearly macroscopically visible at dissection. In contrast, among the three animals treated with BAPN, two did not show tumors at dissection and one showed a single focused tumour macroscopically.

To determine whether or not BAPN was acting at least in part through the senescence response, we directly assayed the SA-β-Gal activity over pancreas samples from mice treated or not with the BAPN. Interestingly, mice treated with BAPN displayed a strong activity whereas the non treated ones were negative.

Therefore, compounds inducing irreversible senescence such as BAPN exert strong anti-tumoral activities.

### SUMMARY OF THE INVENTION

A first object of the present invention is an *in vitro* method for identifying a compound having a tumour growth blocking activity by inducing irreversible senescence in tumour cells comprising the following steps:
- Providing a candidate compound,
- Contacting immortalized mammalian cells comprising an inducible oncogene with both an inducer of said oncogene thereby provoking cellular senescence and with said candidate compound,
- Determining whether said candidate compound inhibits escape from cellular senescence wherein a candidate compound inhibiting escape from cellular senescence is a compound having a tumour growth blocking activity.

Preferably, the immortalized mammalian cells are immortalized human cells selected from the group consisting of Mammary Epithelial Cells, Bronchial Epithelial Cells, Epidermal Keratinocytes, Melanocytes, Prostate Epithelial Cells and Renal Epithelial Cells.

Advantageously, the mammalian cells have been immortalized by constitutive expression of a telomerase (TERT) in said cells.

In preferred embodiments, the inducible oncogene is selected from the group consisting of MEK (MEK:ER), Ras (Ras:ER) and Raf (Raf:ER).

It is preferred that the inducer of the inducible oncogene is 4-hydroxytamoxifen (4-OHT).

Cellular senescence is preferably by the expression of a senescence specific marker such as Senescent Associated β Galactosidase activity (SA-β-Gal).

Advantageously, determining whether the candidate compound inhibits escape from cellular senescence is performed by determining cellular proliferation after removal of the oncogene inducer, wherein a candidate compound inhibiting cellular proliferation is a compound inhibiting escape from cell senescence.

According to a preferred embodiment of the invention, a candidate compound inhibiting escape from cellular senescence is a compound having a tumour growth blocking activity on primary tumour growth and metastatic tumour growth.

According to another preferred embodiment of the present invention, a candidate compound inhibiting escape from cellular senescence is a compound having a tumour growth blocking activity in pancreatic cancer, in breast cancer, melanoma, prostate cancer, kidney cancer, liver cancer, skin cancer and/or glioblastoma.

Another object of the present invention is a composition comprising an inhibitor of Lysyl Oxidase (LOX) selected from beta-aminoproprionitrile (BAPN), antibodies inhibiting human Lysyl oxidase activity, short hairpin RNA inhibiting expression of human Lysyl Oxidase gene and small interfering RNA inhibiting the expression of human Lysyl Oxidase, for use in treatment of primary tumours in Pancreatic Ductal Adenocarcinoma (PDAC).

### DETAILED DESCRIPTION OF THE INVENTION

The present invention is related to new methods for identifying compounds for treating cancers which induce an irreversible senescence in cancer cells. More particularly, the present invention provides methods for screening and identifying candidate compounds which are able to inhibit escape from cellular senescence.

Cellular senescence is the loss of the ability of cells to undergo divisions or proliferation. In the methods of the present invention, senescence is artificially induced in immortalized mammalian cells by the induction of the expression of an inducible oncogene. Induction of the oncogene leads to cell hyper-proliferation which in turn provokes cellular senescence. Surprisingly, it has been found that when the oncogene is no longer induced, the immortalized cells escape cellular senescence and cell proliferation is restarted. Candidate compounds have been identified which prevent restoration of cellular proliferation by the inhibition of escape from cellular senescence. These compounds have been proven to inhibit tumour growth *in vivo.*

A first object of the present invention is an *in vitro* method for identifying a compound having a tumour growth blocking activity by inducing irreversible senescence in tumour cells comprising the following steps:
a) Providing a candidate compound,
b) Contacting immortalized mammalian cells comprising an inducible oncogene with both an inducer of said oncogene thereby provoking cellular senescence and with a candidate compound,
c) Determining whether said candidate compound inhibits escape from cellular senescence wherein a candidate compound inhibiting escape from cellular senescence is a compound having a tumour growth blocking activity.

As used herein, the term "compound" means any chemical or biological entity. The methods of the present invention provide for screening of any candidate compound to identify or select compounds which specifically inhibit tumour growth. The methods of the present invention are suitable for high through put screening of candidate compounds for anti-tumoral activity. Candidate compounds may have or are suspected of having an anti-tumoral activity. The present invention provides for high through put methods for identifying compounds having anti-tumoral activity among candidate compounds.

The methods of the present invention are performed with mammalian cells which a have been immortalized. These cells may proliferate indefinitely but they do not exhibit a tumoral phenotype. Importantly, these immortalized mammalian cells have retained their capacity to enter cellular senescence in response to the induction of an oncogene. In normal cells, cellular senescence functions as failsafe machinery which prevents the onset of cancer.

Any suitable immortalized mammalian cells may be used in the methods of the present invention. In preferred embodiments the mammalian cells are human cells, more preferably human epithelial cells and even more preferably cells selected in the group consisting of Mammary Epithelial Cells, Bronchial Epithelial Cells, Epidermal Keratinocytes, Melanocytes, Prostate Epithelial Cells and Renal Epithelial Cells.

Mammalian cells are available from various commercial sources and may for example be purchased from LONZA® (HMEC-Mammary Epithelial Cells CC-2551, NHBE-Bronchial Epi Cells CC-2540, NHEK-epidermal keratinocytes CC-2501, Melanocytes CC-2586, PrEC-Prostate Epithelial Cells CC-2555, HRE-Renal Epithelial Cells CC-2556).

Typically mammalian cells may be immortalized by the constitutive expression of a telomerase gene (TERT) in these cells (Kiyono et al., 1998; Lee et al., 2004; Piao et al., 2005; Kogan et al., 2006; Wieser et al., 2008). A telomerase gene from the same species or from another species may be constitutively expressed in the mammalian cells. Human cells may for example constitutively express the human telomerase gene GenBank No. NM_198253.2 orNM_198255.2 or any suitable mammalian telomerase gene.

The immortalized cells used to perform the methods of the present invention have been transfected with an inducible oncogene.

As used herein, the term "oncogene" means a gene which upon activation or over-expression will cause cells to proliferate. The term "inducible" means that the expression or the activity of the oncogene is conditionally induced by an exogenous inducer. Various inducible oncogenes and genetic constructs for infecting mammalian cells have been described in the scientific literature.

In preferred embodiments, the inducible oncogene is MEK:ER (the hormone-binding domain of the estrogen receptor (ER) is fused to the C terminus of constitutively activated Mek1. The MEK:ER construct has been described in the literature (Greulich and Erikson, 1998; Acosta et al., 2008 and Humbert et al., 2009).

In other embodiments, the inducible oncogene is selected from RAS:ER (Young et al., 2009; Barradas et al., 2009) and RAF:ER (Agger et al., 2009; Christoffersen et al., 2010).

The inducer starts oncogene expression or oncogene activity. In preferred embodiments the MEK:ER gene is activated by 4-hydroxytamoxifen (4-OHT) inducer.

Induction of the oncogene in the immortalized mammalian cells induces cell hyper-proliferation which in turn leads to cellular senescence. Induction of the oncogene may be performed according to any known method. Typically, the mammalian cells are contacted with an appropriate amount of inducer by addition of the inducer to the culture medium for 1-5 days.

Cellular senescence is a well-known phenomenon described in the literature. Cellular markers that are specifically expressed in cells undergoing senescence have been described. In preferred embodiments, cellular senescence is characterized by the expression of a senescence specific marker such as Senescent Associated β Galactosidase activity (SA-β-Gal). Appropriate tests are available to detect SA-β-Gal activity in cells and to detect cellular senescence. Cellular senescence is also characterized by a cell growth or cellular proliferation/division arrest.

It has now been observed that after removal of the inducer activating the oncogene, cells spontaneously escape cellular senescence and cellular proliferation/division starts again. Escape from cellular senescence and restoration of cellular proliferation is generally observed 1-5 days after removal of the oncogene inducer.

This escape from cellular senescence may be inhibited and a candidate compound may therefore be tested or assessed for its capacity to inhibit escape from cellular senescence. The ability of a candidate compound is typically assessed by measuring cellular proliferation after removal of the oncogene inducer. A candidate compound inhibiting cellular proliferation after removal of the oncogene inducer is a compound inhibiting escape from cellular senescence.

Senescence is one of the cellular mechanisms which prevent the onset of cancer. Compounds inhibiting cellular escape from senescence are therefore useful for treating tumours. In preferred embodiments, a candidate compound inhibiting escape from cell senescence is a compound having a tumour growth blocking activity on primary tumour growth and metastatic tumour growth.

More preferred, a candidate compound inhibiting escape from cell senescence is a compound having a tumour growth blocking activity in pancreatic cancer, in breast cancer, melanoma, prostate cancer, kidney cancer, liver cancer, skin cancer and/or in glioblastoma.

The compounds identified by the methods of the present invention are useful for the treatment of cancers, preferably for the treatment of primary tumours and even more preferably for the treatment of pancreatic cancer.

The methods of the present invention have been applied to identify a candidate compound, BAPN, inhibiting escape from cellular senescence. This candidate compound, BAPN (beta-aminoproprionitrile) had already been described for the treatment of metastatic tumours but no effect had been detected on primary tumours in several reports. BAPN is a well known inhibitor of Lysyl Oxidase. Inhibitors of Lysyl Oxidase (LOX) such as BAPN, antibodies inhibiting Lysyl oxidase activity and Short Hairpin RNA inhibiting Lysyl oxidase gene expression have been described for treatment of metastatic tumours.

BAPN gives a positive result in the screening methods provided by the present invention which identify compounds which block tumour growth by inducing irreversible senescence. The activity of BAPN on primary tumours was therefore tested in a mouse model of pancreatic cancer which revealed a strong activity of BAPN on primary tumours in pancreatic cancer. These results validate the screening methods described in the present invention and confirm that the methods of the present invention provide an efficient tool to identify compounds for treatment of cancers and in particular for treatment of primary tumours.

The present invention is also related to compositions comprising an inhibitor of Lysyl Oxidase (LOX) for use in treatment of primary tumours in pancreatic cancer.

Inhibitors of Lysyl oxidase have for example been described in US 5182297, US 5059714, US 5021456, US4965288, US4943593, US 2008/0293936 and EP 1497269.

In preferred embodiments, the inhibitor of LOX is selected from beta-aminoproprionitrile (BAPN), antibodies inhibiting human Lysyl oxidase activity and interfering RNA inhibiting expression of human Lysyl Oxidase gene. Other inhibitors of LOX described in the state of the art may be used in the compositions of the present invention.

Inhibition of the expression or activity of the LOX gene may be performed by using at least an interfering RNA. The interfering RNA molecules are used to specifically target messenger RNA (mRNA): the interfering RNA hybridizes with the mRNA and consequently inhibits the translation of the corresponding protein, either by simple steric hindrance or by promoting mRNA cleavage.

The interfering RNA can be chosen from a plurality of forms, such as: antisense RNA, double-stranded RNA, "small interfering" RNA (siRNA), "Small Hairpin" RNA (shRNA) or ribosomal RNA.

The siRNA, for "Small Interfering RNA" are short sequences of around 15 to 30 base pairs. They include a first strand and an additional strand identical to the targeted region of the RNA of the target gene.

The shRNA for "Small Hairpin RNA" are double-stranded RNA produced by a cloned sequence in an expression vector, coding for a RNA molecule that will adopt a hairpin shape after cleavage by the Dicer and loquacious complex.

The design and preparation of interfering RNA and their use are well known and widely described in numerous publications.

In preferred embodiments,

the compositions are for use in treatment of primary tumours in Pancreatic Ductal Adenocarcinoma (PDAC).

The present invention also provides pharmaceutical compositions comprising:
a) an effective amount of an inhibitor of LOX, and
b) a pharmaceutically acceptable carrier, which may be inert or physiologically active.

As used herein, "pharmaceutically-acceptable carriers" includes any and all solvents, dispersion media, coatings, antibacterial and antifungal agents, and the like that are physiologically compatible. Examples of suitable carriers, diluents and/or excipients include one or more of water, saline, phosphate buffered saline, dextrose, glycerol, ethanol, and the like, as well as combination thereof. In many cases, it will be preferable to include isotonic agents, such as sugars, polyalcohols, or sodium chloride in the composition. In particular, relevant examples of suitable carrier include: (1) Dulbecco's phosphate buffered saline, pH - 7.4, containing or not containing about 1 mg/ml to 25 mg/ml human serum albumin, (2) 0.9% saline (0.9% w/v sodium chloride (NaCl)), and (3) 5% (w/v) dextrose; and may also contain an antioxidant such as tryptamine and a stabilizing agent such as Tween 20.

The pharmaceutical compositions encompassed by the present invention may also contain a further therapeutic agent for the treatment of cancers.

The compositions of the invention may be in a variety of forms. These include for example liquid, semi-solid, and solid dosage forms, but the preferred form depends on the intended mode of administration and therapeutic application. Typical preferred compositions are in the form of injectable or infusible solutions. The preferred mode of administration is parenteral (e.g. intravenous, intramuscular, intraperinoneal, subcutaneous). In a preferred embodiment, the compositions of the invention are administered intravenously as a bolus or by continuous infusion over a period of time. In another preferred embodiment, they are injected by intramuscular, subcutaneous, intra-articular, intrasynovial, intratumoral, peritumoral, intralesional, or perilesional routes, to exert local as well as systemic therapeutic effects.

Sterile compositions for parenteral administration can be prepared by incorporating the LOX inhibitor in the required amount in the appropriate solvent, followed by sterilization by microfiltration. As solvent or vehicle, there may be used water, saline, phosphate buffered saline, dextrose, glycerol, ethanol, and the like, as well as combination thereof. In many cases, it will be preferable to include isotonic agents, such as sugars, polyalcohols, or sodium chloride in the composition. These compositions may also contain adjuvants, in particular wetting, isotonizing, emulsifying, dispersing and stabilizing agents. Sterile compositions for parenteral administration may also be prepared in the form of sterile solid compositions which may be dissolved at the time of use in sterile water or any other injectable sterile medium.

The compositions comprising a LOX inhibitor may also be orally administered. As solid compositions for oral administration, tablets, pills, powders (gelatine capsules, sachets) or granules may be used. In these compositions, the active ingredient according to the invention is mixed with one or more inert diluents, such as starch, cellulose, sucrose, lactose or silica, under an argon stream. These compositions may also comprise substances other than diluents, for example one or more lubricants such as magnesium stearate or talc, a coloring, a coating (sugar-coated tablet) or a glaze.

As liquid compositions for oral administration, there may be used pharmaceutically acceptable solutions, suspensions, emulsions, syrups and elixirs containing inert diluents such as water, ethanol, glycerol, vegetable oils or paraffin oil. These compositions may comprise substances other than diluents, for example wetting, sweetening, thickening, flavoring or stabilizing products.

The doses depend on the desired effect, the duration of the treatment and the route of administration used.

The invention is also related to the use of an inhibitor of LOX such as BAPN for the manufacture of a medicament for treatment of primary pancreatic tumours.

Also described are methods for treating primary tumours in cancers including administering an effective amount of a LOX inhibitor such as BAPN to a human or to a patient in need thereof, preferably methods for treatment of primary tumours in pancreatic cancer and more particularly methods for treatment of Pancreatic Ductal Adenocarcinoma (PDAC).

### FIGURES

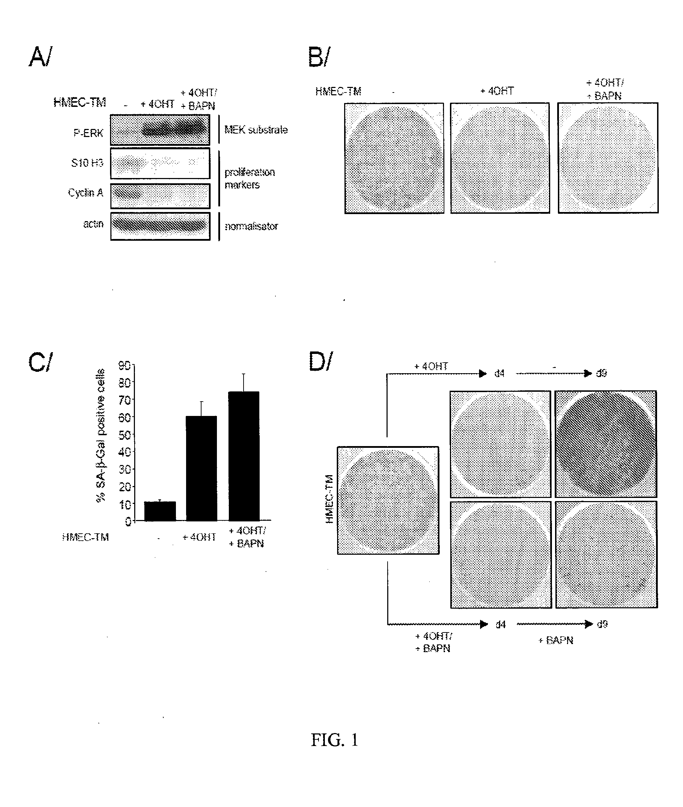

Figure 1: A model to screen for compounds stabilising the senescence response. HMEC-TM were treated by 500 nM 4-OHT with or without BAPN every days during 3 days. Next day, various analyses were performed. (A) Cellular extracts were prepared and analysed by immunoblotting using antibodies targeting phospho-ERK, Cyclin A, phosphoS10 H3, and actin. (B) The cells were fixed and crystal violet stained. (C) An SA-β-Gal analysis was performed and percentage of labelled cells was calculated. (D) After 4-OHT treatment, media of cells were changed every day with or without BAPN. After 9 days, cells were fixed and crystal violet stained.
Figure 2: Lox inhibition by BAPN treatment blocks tumor growth.

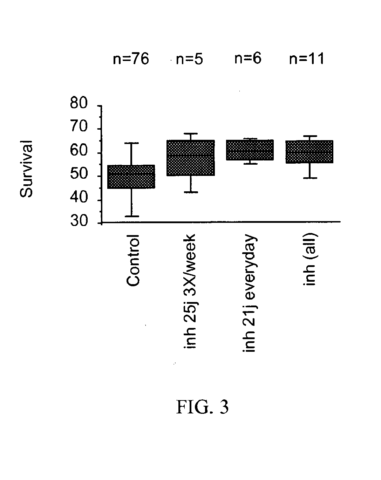

Figure 3: Lox inhibition increases survival.

Mice were euthanized when they have lost 10% of their weight. Mice were treated every 2 days, 25 days after birth (BAPN1) or every days, 21 days after birth (BAPN2) up to their sacrifice.

### EXAMPLES

### Materials and Methods

### Cell Culture

Human mammary epithelial cells (HMEC) (Lonza) were cultured in MEBM media (Promocell) and gentamycin (invitrogen). Virus producing GP 293 cells (clontech) were cultured in DMEM media (invitrogen) supplemented with 10 % FBS (Hyclone) and gentamycin.

### Reagents

Four-Hydroxytamoxifen (4-OHT) (Sigma) was used at a final concentration of 500 nM. Three aminopropionitrile (fumarate salt) (Sigma) was used at a final concentration of 125 ng/µL for *in vitro* analysis.

### Transfection and infection

GP 293 cells were transfected using PEI reagents according to manufacturer's recommendations (Euromedex). Three days after transfection, viral supernatant mixed with fresh media (1/2) and polybrene (final concentration at 8 µg/mL) was used to infect HMEC target cells.

### Antibodies and immunoblot

Cell extracts were prepared and analysed as described in (REF). The following antibodies were used: anti-phosphoERK (9101, Cell Signaling), anti-histone3phosphoS10 (ab14955, Abcam), anti-cyclinA (H432, Sigma) and anti-actin (A5316, Sigma).

### Colony formation assays

Thirty thousand cells were seeded in 6-well plates and treated or not with various compounds as indicated for each experiments. At the end of the experiments, cells were fixed with PFA 4% for 15 minutes, wash with water and fixed by with a crystal violet solution (Sigma).

### Mice engineering and treatment

Breeding Pdx1-Cre;Ink4a/Arf-KO^{lox/lox} (no phenotype) with Kras-CA^{lox/+};Ink4a/Arf-KO^{lox/lox} (no phenotype) individuals allows us to "routinely" generate Pdx1-Cre;Kras-CA^{lox/+};Ink4a/Arf-KO^{lox/lox} animals (representing 25% of the all progenies according to expected Mendelian inheritance), which develop macroscopic pancreatic cancer at the frequency of 100% by the age of 6-7 weeks. As early as the age of 3 weeks, obtained mice were treated by intraperitoneal injection of 100 mg/Kg of BAPN (dissolved in saline solution) or with the vehicles. These injections were performed three times per week during 3 weeks. At 6-7 weeks, the mice were euthanized and their pancreas removed for histological analysis.

### SA-β-Gal analysis

Senescent associated-β- Galactosidase activity was performed on HMEC (cells) or pancreatic sections fixed and stained as recommended by the manufaturer's (senescence beta-galactosidase kit, Cell Signaling).

### Results

### A cellular model to screen for new drugs based on their activities over senescence

Immortalization of normal HMEC was achieved by retroviral infection with TERT expressing vectors. Immortalized HMEC-TERT were transduced with a retroviral vector encoding an inducible MEK oncogene (MEK:ER) to obtain HMEC-TERT-MEK:ER (HMEC-TM). HMEC-TM cells were treated with 500nM 4-OHT every day during 3 days. At day 4, MEK activity was examined by looking at the phosphorylation of its substrate ERK. As expected, 4-OHT treatment induced MEK activity as this treatment resulted in an increase in phospho-ERK (Figure 1A). This activation resulted in a growth arrest according to the colony formation assay (Figure 1B) and to the decrease of expression of proliferation markers cyclin A and phospho-Ser10 histone 3 (Figure 1A). This growth arrest is due to senescence as these cells were positive for the senescence marker SA-β-Gal activity (Figure 1C).

Senescence is considered as an irreversible mechanism, nevertheless during the transformation process or during chemotherapeutic treatment, cells escape these processes to continue to grow. We wondered whether or not the HMEC-TM cells were able to spontaneously escape senescence when we stopped the 4-OHT treatment. The 4-OHT treatment was then stopped when the cells were in the senescence state (after 3 days of treatment). Interestingly, in this cellular model cells spontaneously escaped senescence as they were able to form numerous colonies after 9 days (Figure 1D).

As escape from senescence is a major problem in tumor response, we propose that this cellular model could be the basis of specific screens to isolate new drugs able to re-enforce and/or stabilize senescence response by avoiding spontaneous escape.

### Lox inhibition by the BAPN inhibitor blocks (pancreatic) tumor growth

As a proof of concept, we were looking for a chemical compound able to re-enforce and/or stabilize senescence in HMEC-TM. We found that BAPN, a well-known chemical inhibitor of lysyl oxidase activity, was able to do so. Interestingly, BAPN treatment did not modify MEK activation according to the level of phosphorylation of its substrate ERK (Figure 1A). Nevertheless, BAPN treatment resulted in a slight increase of senescence: cyclin A level (Figure 1A), colony assay formation (Figure 1B) and percentage of SA-β-Gal positive cells (Figure 1C). Importantly, beside the re-enforcement of the senescence response, BAPN treatment completely blocks senescence escape observed in non treated cells (Figure ID). These results confirm the suitability of this model to screen for compounds that re-enforces senescence and blocks spontaneous senescence escape.

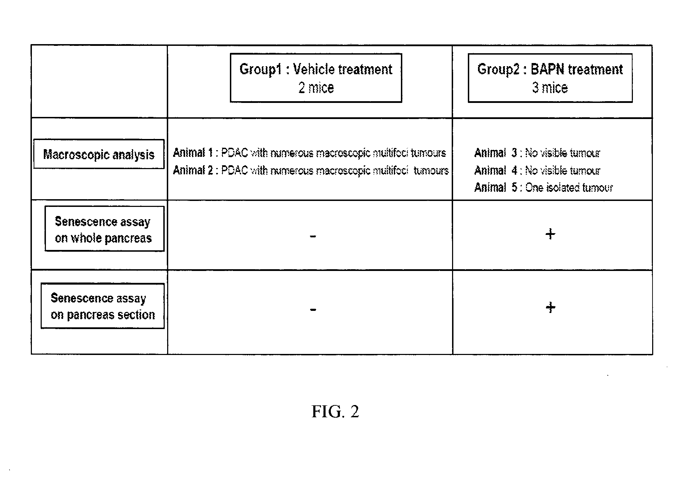

As BAPN is a powerful inhibitor to re-enforce and stabilize senescence *in vitro,* we explored its anti-tumoral activity in primary tumors. We wanted to know, as our *in vitro* results suggested, whether or not the LOX inhibition by BAPN treatment could inhibit primary tumor growth. To this end we used a genetically modified mouse expressing a mutated K-RAS and knocked out for the Ink4A/Arf locus. These mice develop aggressive pancreatic tumors in few weeks after birth (Aguirre et al., 2003). To test a potential effect of Lox inhibition by BAPN, we treated these mice by intra peritoneal injection of vehicle (2 mice) or BAPN (3 mice). 6-7 weeks after birth, mice were sacrificed and pancreatic tumors examined. As expected, the two control mice treated with "vehicle" showed numerous mutifoci pancreatic tumors clearly macroscopically visible at dissection. In contrast, among the three animals treated with BAPN, two did not show obvious tumors at dissection and one showed a single focused tumour. To determine whether or not BAPN was acting at least in part through the senescence response, we directly assayed the SA-β-Gal activity over pancreas samples from mice treated or not with the BAPN. Interestingly, mice treated with BAPN displayed a strong activity whereas the non treated ones were negative (Figure 2). Lox inhibition by BAPN treatment exerts strong anti-tumoral activities.

In conclusion, we have described a new cellular model and method to screen for new drugs that control the senescence response and in particular are able to block spontaneous senescence escape. The concept of the use of this cellular model to screen for anti-tumoral compounds was validated by the finding that LOX inhibition by BAPN treatment completely blocked senescence escape *in vitro* in our cellular model. Interestingly, in an aggressive model of pancreatic tumors, BAPN treatment blocked tumor growth at least in part through senescence induction. According to the general involvement of the loss of senescence response in various kinds of tumors, we expect that LOX inhibition by BAPN could constitute an anti-tumoral strategy in numerous kinds of tumors.

### REFERENCES

1. Acosta,J.C., O'Loghlen,A., Banito,A., Guijarro,M.V., Augert,A., Raguz,S., Fumagalli,M., Da Costa,M., Brown,C., Popov,N., Takatsu,Y., Melamed,J., d'Adda di Fagagna,F., Bernard,D., Hernando,E., and Gil,J. (2008). Chemokine Signaling via the CXCR2 Receptor Reinforces Senescence. Cell, 133, 1006-1018.
2. Agger,K., Cloos,P.A., Rudkjaer,L., Williams,K., Andersen,G., Christensen,J., and Helin,K. (2009). The H3K27me3 demethylase JMJD3 contributes to the activation of the INK4A-ARF locus in response to oncogene- and stress-induced senescence. Genes Dev., 23, 1171-1176.
3. Aguirre,A.J., Bardeesy,N., Sinha,M., Lopez,L., Tuveson,D.A., Horner,J., Redston,M.S., and DePinho,R.A. (2003). Activated Kras and Ink4a/Arf deficiency cooperate to produce metastatic pancreatic ductal adenocarcinoma. Genes Dev., 17, 3112-3126.
4. Bardeesy,N., Sharpless,N.E., DePinho,R.A., and Merlino,G. (2001). The genetics of pancreatic adenocarcinoma: a roadmap for a mouse model. Semin. Cancer Biol., 11, 201-218.
5. Barradas,M., Anderton,E., Acosta,J.C., Li,S., Banito,A., Rodriguez-Niedenfuhr,M., Maertens,G., Banck,M., Zhou,M.M., Walsh,M.J., Peters,G., and Gil,J. (2009). Histone demethylase JMJD3 contributes to epigenetic control of INK4a/ARF by oncogenic RAS. Genes Dev., 23, 1177-1182.
6. Braig,M., Lee,S., Loddenkemper,C., Rudolph,C., Peters,A.H., Schlegelberger,B., Stein,H., Dorken,B., Jenuwein,T., and Schmitt,C.A. (2005). Oncogene-induced senescence as an initial barrier in lymphoma development. Nature, 436, 660-665.
7. Chen,Z., Trotman,L.C., Shaffer,D., Lin,H.K., Dotan,Z.A., Niki,M., Koutcher,J.A., Scher,H.I., Ludwig,T., Gerald,W., Cordon-Cardo,C., and Paolo Pandolfi,P. (2005). Crucial role of p53-dependent cellular senescence in suppression of Pten-deficient tumorigenesis. Nature, 436, 725-730.
8. Christoffersen,N.R., Shalgi,R., Frankel,L.B., Leucci,E., Lees,M., Klausen,M., Pilpel,Y., Nielsen,F.C., Oren,M., and Lund,A.H. (2010). p53-independent upregulation of miR-34a during oncogene-induced senescence represses MYC. Cell Death. Differ., 17,236-245.
9. Collado,M., Gil,J., Efeyan,A., Guerra,C., Schuhmacher,A.J., Barradas,M., Benguria,A., Zaballos,A., Flores,J.M., Barbacid,M., Beach,D., and Serrano,M. (2005). Tumour biology: Senescence in premalignant tumours. Nature, 436, 642.
10. Dimri,G.P., Lee,X., Basile,G., Acosta,M., Scott,G., Roskelley,C., Medrano,E.E., Linskens,M., Rubelj,I., and Pereira-Smith,O. (1995). A biomarker that identifies senescent human cells in culture and in aging skin in vivo. Proc Natl Acad Sci U S A, 92, 9363-9367.
11. Erler,J.T., Bennewith,K.L., Cox,T.R., Lang,G., Bird,D., Koong,A., Le,Q.T., and Giaccia,A.J. (2009). Hypoxia-induced lysyl oxidase is a critical mediator of bone marrow cell recruitment to form the premetastatic niche. Cancer Cell, 15, 35-44.
12. Erler,J.T., Bennewith,K.L., Nicolau,M., Dornhofer,N., Kong,C., Le,Q.T., Chi,J.T., Jeffrey,S.S., and Giaccia,A.J. (2006). Lysyl oxidase is essential for hypoxia-induced metastasis. Nature, 440, 1222-1226.
13. Greulich,H. and Erikson,R.L. (1998). An analysis of Mek1 signaling in cell proliferation and transformation. J. Biol. Chem., 273, 13280-13288.
14. Hingorani,S.R., Petricoin,E.F., Maitra,A., Rajapakse,V., King,C., Jacobetz,M.A., Ross,S., Conrads,T.P., Veenstra,T.D., Hitt,B.A., Kawaguchi,Y., Johann,D., Liotta,L.A., Crawford,H.C., Putt,M.E., Jacks,T., Wright,C.V., Hruban,R.H., Lowy,A.M., and Tuveson,D.A. (2003). Preinvasive and invasive ductal pancreatic cancer and its early detection in the mouse. Cancer Cell, 4, 437-450.
15. Humbert,N., Martien,S., Augert,A., Da Costa,M., Mauen,S., Abbadie,C., de Launoit,Y., Gil,J., and Bernard,D. (2009). A genetic screen identifies topoisomerase 1 as a regulator of senescence. Cancer Res, 69, 4101-4106.
16. Kiyono,T., Foster,S.A., Koop,J.I., McDougall,J.K., Galloway,D.A., and Klingelhutz,A.J. (1998). Both Rb/p16INK4a inactivation and telomerase activity are required to immortalize human epithelial cells. Nature, 396, 84-88.
17. Kogan,I., Goldfinger,N., Milyavsky,M., Cohen,M., Shats,I., Dobler,G., Klocker,H., Wasylyk,B., Voller,M., Aalders,T., Schalken,J.A., Oren,M., and Rotter,V. (2006). hTERT-immortalized prostate epithelial and stromal-derived cells: an authentic in vitro model for differentiation and carcinogenesis. Cancer Res., 66, 3531-3540.
18. Lee,K.M., Choi,K.H., and Ouellette,M.M. (2004). Use of exogenous hTERT to immortalize primary human cells. Cytotechnology, 45, 33-38.
19. Piao,C.Q., Liu,L., Zhao,Y.L., Balajee,A.S., Suzuki,M., and Hei,T.K. (2005). Immortalization of human small airway epithelial cells by ectopic expression of telomerase. Carcinogenesis, 26, 725-731.
20. Wieser,M., Stadler,G., Jennings,P., Streubel,B., Pfaller,W., Ambros,P., Riedl,C., Katinger,H., Grillari,J., and Grillari-Voglauer,R. (2008). hTERT alone immortalizes epithelial cells of renal proximal tubules without changing their functional characteristics. Am. J. Physiol Renal Physiol, 295, F1365-F1375.
21. Young,A.R., Narita,M., Ferreira,M., Kirschner,K., Sadaie,M., Darot,J.F., Tavare,S., Arakawa,S., Shimizu,S., Watt,F.M., and Narita,M. (2009). Autophagy mediates the mitotic senescence transition. Genes Dev., 23, 798-803.
22. Bondareva et al. (2009), Plos one, vol.4, no. 5

### PATENT REFERENCES

WO 2007/126457
WO2009/017833
EP 1497269
US 2008/0293936
US 4943593
US 4965288
US 5021456
US 5059714
US 5182297

## Claims

1. An *in vitro* method for identifying a compound having a tumour growth blocking activity by inducing irreversible senescence in tumour cells comprising the following steps:
a) Providing a candidate compound,
b) Contacting immortalized mammalian cells comprising an inducible oncogene with both an inducer of said oncogene thereby provoking cellular senescence and with said candidate compound,
c) Determining whether said candidate compound inhibits escape from cellular senescence wherein a candidate compound inhibiting escape from cellular senescence is a compound having a tumour growth blocking activity.

2. An *in vitro* method for identifying a compound having a tumour growth blocking activity by inducing irreversible senescence in tumour cells according to claim 1 wherein the immortalized mammalian cells are immortalized human cells selected from the group consisting of Mammary Epithelial Cells, Bronchial Epithelial Cells, Epidermal Keratinocytes, Melanocytes, Prostate Epithelial Cells and Renal Epithelial Cells.

3. An *in vitro* method for identifying a compound having a tumour growth blocking activity by inducing irreversible senescence in tumour cells according to anyone of claims 1-2 wherein the mammalian cells have been immortalized by constitutive expression of a telomerase (TERT) in said cells.

4. An *in vitro* method for identifying a compound having a tumour growth blocking activity by inducing irreversible senescence in tumour cells according to anyone of claims 1-3 wherein the inducible oncogene is selected from the group consisting of MEK (MEK:ER), Ras (Ras:ER) and Raf (Raf:ER).

5. An *in vitro* method for identifying a compound having a tumour growth blocking activity by inducing irreversible senescence in tumour cells according to anyone of claims 1-4 wherein the inducer of the inducible oncogene is 4-hydroxytamoxifen (4-OHT).

6. An *in vitro* method for identifying a compound having a tumour growth blocking activity by inducing irreversible senescence in tumour cells according to anyone of claims 1-5 wherein cellular senescence is **characterized by** the expression of a senescence specific marker such as Senescent Associated β Galactosidase activity (SA-β-Gal).

7. An *in vitro* method for identifying a compound having a tumour growth blocking activity by inducing irreversible senescence in tumour cells according to anyone of claims 1-6 wherein determining whether the candidate compound inhibits escape from cellular senescence is assessed by determining cellular proliferation after removal of the oncogene inducer and wherein a candidate compound inhibiting cellular proliferation is a compound inhibiting escape from cell senescence.

8. An *in vitro* method for identifying a compound having a tumour growth blocking activity by inducing irreversible senescence in tumour cells according to anyone of claims 1-7 wherein a candidate compound inhibiting escape from cellular senescence is a compound having a tumour growth blocking activity on primary tumour growth and metastatic tumour growth.

9. An *in vitro* method for identifying a compound having a tumour growth blocking activity by inducing irreversible senescence in tumour cells according to anyone of claims 1-8 wherein a candidate compound inhibiting escape from cellular senescence is a compound having a tumour growth blocking activity in pancreatic cancer, in breast cancer, melanoma, prostate cancer, kidney cancer, liver cancer, skin cancer, glioblastoma.

10. Composition comprising an inhibitor of Lysyl Oxidase (LOX) selected from beta-aminoproprionitrile (BAPN), antibodies inhibiting human Lysyl oxidase activity and interfering RNA inhibiting the expression of human Lysyl Oxidase, for use in treatment of primary tumours in cancer in Pancreatic Ductal Adenocarcinoma (PDAC).

## Patentansprüche

1. *In vitro*-Verfahren zum Identifizieren einer Verbindung, welche eine das Tumorwachstum blockierende Aktivität aufweist, indem irreversible Seneszenz in Tumorzellen induziert wird, welches die folgenden Schritte umfasst:
a) Bereitstellen einer Kandidaten-Verbindung,
b) Inkontaktbringen von immortalisierten Säugetierzellen, welche ein induzierbares Onkogen umfassen, sowohl mit einem Induktionsmittel des Onkogens, wodurch zelluläre Seneszenz hervorgerufen wird, als auch mit der Kandidaten-Verbindung,
c) Bestimmen, ob die Kandidaten-Verbindung ein Entkommen aus der zellulären Seneszenz inhibiert, wobei eine Kandidaten-Verbindung, die ein Entkommen aus der zellulären Seneszenz inhibiert, eine Verbindung, welche eine das Tumorwachstum blockierende Aktivität aufweist, ist.

2. *In vitro*-Verfahren zum Identifizieren einer Verbindung, welche eine das Tumorwachstum blockierende Aktivität aufweist, indem irreversible Seneszenz in Tumorzellen induziert wird, nach Anspruch 1, wobei die immortalisierten Säugetierzellen immortalisierte humane Zellen, ausgewählt aus der Gruppe bestehend aus Mammaepithelzellen, bronchialen Epithelzellen, epidermalen Keratinozyten, Melanozyten, Prostataepithelzellen und Nierenepithelzellen, sind.

3. *In vitro*-Verfahren zum Identifizieren einer Verbindung, welche eine das Tumorwachstum blockierende Aktivität aufweist, indem irreversible Seneszenz in Tumorzellen induziert wird, nach einem der Ansprüche 1-2, wobei die Säugetierzellen durch konstitutive Expression einer Telomerase (TERT) in den Zellen immortalisiert worden sind.

4. *In vitro*-Verfahren zum Identifizieren einer Verbindung, welche eine das Tumorwachstum blockierende Aktivität aufweist, indem irreversible Seneszenz in Tumorzellen induziert wird, nach einem der Ansprüche 1-3, wobei das induzierbare Onkogen ausgewählt wird aus der Gruppe bestehend aus MEK (MEK:ER), Ras (Ras:ER) und Raf (Raf:ER).

5. *In vitro*-Verfahren zum Identifizieren einer Verbindung, welche eine das Tumorwachstum blockierende Aktivität aufweist, indem irreversible Seneszenz in Tumorzellen induziert wird, nach einem der Ansprüche 1-4, wobei das Induktionsmittel des induzierbaren Onkogens 4-Hydroxytamoxifen (4-OHT) ist.

6. *In vitro*-Verfahren zum Identifizieren einer Verbindung, welche eine das Tumorwachstum blockierende Aktivität aufweist, indem irreversible Seneszenz in Tumorzellen induziert wird, nach einem der Ansprüche 1-5, wobei zelluläre Seneszenz durch die Expression von einem für Seneszenz spezifischen Marker, wie von mit Seneszenz assoziierte β-Galaktosidase-Aktivität (SA-β-Gal), gekennzeichnet ist.

7. *In vitro*-Verfahren zum Identifizieren einer Verbindung, welche eine das Tumorwachstum blockierende Aktivität aufweist, indem irreversible Seneszenz in Tumorzellen induziert wird, nach einem der Ansprüche 1-6, wobei das Bestimmen, ob die Kandidaten-Verbindung ein Entkommen aus der zellulären Seneszenz inhibiert, ermittelt wird, indem zelluläre Proliferation nach Entfernung des Onkogen-Induktionsmittels bestimmt wird, und wobei eine Kandidaten-Verbindung, welche zelluläre Proliferation inhibiert, eine Verbindung ist, welche ein Entkommen aus der zellulären Seneszenz inhibiert.

8. *In vitro*-Verfahren zum Identifizieren einer Verbindung, welche eine das Tumorwachstum blockierende Aktivität aufweist, indem irreversible Seneszenz in Tumorzellen induziert wird, nach einem der Ansprüche 1-7, wobei eine Kandidaten-Verbindung, welche ein Entkommen aus der zellulären Seneszenz inhibiert, eine Verbindung ist, welche eine das Tumorwachstum blockierende Aktivität auf primäres Tumorwachstum und metastasierendes Tumorwachstum aufweist.

9. *In vitro*-Verfahren zum Identifizieren einer Verbindung, welche eine das Tumorwachstum blockierende Aktivität aufweist, indem irreversible Seneszenz in Tumorzellen induziert wird, nach einem der Ansprüche 1-8, wobei eine Kandidaten-Verbindung, welche ein Entkommen aus der zellulären Seneszenz inhibiert, eine Verbindung ist, welche eine das Tumorwachstum blockierende Aktivität bei Bauchspeicheldrüsenkrebs, bei Brustkrebs, Melanomen, Prostatakrebs, Nierenkrebs, Leberkrebs, Hautkrebs, Glioblastomen aufweist.

10. Zusammensetzung, umfassend einen Inhibitor von Lysyloxidase (LOX), ausgewählt aus beta-Aminoproprionitril (BAPN), Antikörpern, welche humane Lysyloxidase-Aktivität inhibieren, und interferierender RNA, welche die Expression von humaner Lysyloxidase inhibiert, zur Verwendung bei einer Behandlung von primären Tumoren bei Krebs bei duktalem Adenokarzinom des Pankreas (Pancreatic Ductal Adenocarcinoma; PDAC).

## Revendications

1. Procédé *in vitro* pour identifier un composé ayant une activité qui bloque la croissance tumorale en induisant une sénescence irréversible dans les cellules tumorales comprenant les étapes suivantes :
a) se procurer un composé candidat,
b) mettre en contact des cellules de mammifères immortalisées comprenant un oncogène inductible avec à la fois un inducteur dudit oncogène pour provoquer ainsi la sénescence cellulaire et avec ledit composé candidat,
c) déterminer si ledit composé candidat inhibe ou pas le mécanisme d'échappement à la sénescence cellulaire dans lequel un composé candidat qui inhibe le mécanisme d'échappement à la sénescence cellulaire est un composé ayant une activité qui bloque la croissance tumorale.

2. Procédé *in vitro* pour identifier un composé ayant une activité qui bloque la croissance tumorale en induisant une sénescence irréversible dans les cellules tumorales selon la revendication 1 dans lequel les cellules de mammifères immortalisées sont des cellules immortalisées humaines choisies dans le groupe constitué par les cellules épithéliales mammaires, les cellules épithéliales bronchiques, les kératinocytes épidermiques, les mélanocytes, les cellules épithéliales prostatiques et les cellules épithéliales rénales.

3. Procédé *in vitro* pour identifier un composé ayant une activité qui bloque la croissance tumorale en induisant une sénescence irréversible dans les cellules tumorales selon l'une quelconque des revendications 1-2 dans lequel les cellules de mammifères ont été immortalisées par l'expression constitutive d'une télomérase (TERT) dans lesdites cellules.

4. Procédé *in vitro* pour identifier un composé ayant une activité qui bloque la croissance tumorale en induisant une sénescence irréversible dans les cellules tumorales selon l'une quelconque des revendications 1-3 dans lequel l'oncogène inductible est choisi dans le groupe constitué par MEK (MEK:ER), Ras (Ras:ER) et Raf (Raf:ER).

5. Procédé *in vitro* pour identifier un composé ayant une activité qui bloque la croissance tumorale en induisant une sénescence irréversible dans les cellules tumorales selon l'une quelconque des revendications 1-4 dans lequel l'inducteur de l'oncogène inductible est le 4-hydroxytamoxifène (4-OHT).

6. Procédé *in vitro* pour identifier un composé ayant une activité qui bloque la croissance tumorale en induisant une sénescence irréversible dans les cellules tumorales selon l'une quelconque des revendications 1-5 dans lequel la sénescence cellulaire est **caractérisée par** l'expression d'un marqueur spécifique de sénescence tel qu'une activité β-galactosidase associée à la sénescence (SA-β-Gal).

7. Procédé *in vitro* pour identifier un composé ayant une activité qui bloque la croissance tumorale en induisant une sénescence irréversible dans les cellules tumorales selon l'une quelconque des revendications 1-6 dans lequel la détermination selon laquelle le composé candidat inhibe ou pas le mécanisme d'échappement à la sénescence cellulaire est évaluée en déterminant la prolifération cellulaire après élimination de l'inducteur d'oncogène et dans lequel un composé candidat qui inhibe la prolifération cellulaire est un composé qui inhibe le mécanisme d'échappement à la sénescence cellulaire.

8. Procédé *in vitro* pour identifier un composé ayant une activité qui bloque la croissance tumorale en induisant une sénescence irréversible dans les cellules tumorales selon l'une quelconque des revendications 1-7 dans lequel un composé candidat qui inhibe le mécanisme d'échappement à la sénescence cellulaire est un composé ayant une activité qui bloque la croissance tumorale sur la croissance tumorale primaire et la croissance tumorale métastasique.

9. Procédé *in vitro* pour identifier un composé ayant une activité qui bloque la croissance tumorale en induisant une sénescence irréversible dans les cellules tumorales selon l'une quelconque des revendications 1-8 dans lequel un composé candidat qui inhibe le mécanisme d'échappement à la sénescence cellulaire est un composé ayant une activité qui bloque la croissance tumorale dans le cancer du pancréas, le cancer du sein, le mélanome, le cancer de la prostate, le cancer du rein, le cancer du foie, le cancer de la peau, le glioblastome.

10. Composition comprenant un inhibiteur de lysyle oxydase (LOX) choisi parmi le bêta-aminopropionitrile (BAPN), les anticorps qui inhibent l'activité lysyle oxydase humaine et l'ARN d'interférence qui inhibe l'expression de la lysyle oxydase humaine, qui peut être utilisée pour traiter les tumeurs primaires cancéreuses dans l'adénocarcinome ductal pancréatique (PDAC).
